Europäisches Patentamt

European Patent Office

Office européen des brevets

(19)

(11) Publication number: **0 304 515 B1**

# (12) EUROPEAN PATENT SPECIFICATION

(45) Date of publication of patent specification: **04.12.91**   (51) Int. Cl.5: **C07C 6/04**, B01J 23/36

(21) Application number: **87307333.2**

(22) Date of filing: **19.08.87**

(54) **A process for the disproportionation of alkenes.**

(43) Date of publication of application:
**01.03.89 Bulletin 89/09**

(45) Publication of the grant of the patent:
**04.12.91 Bulletin 91/49**

(84) Designated Contracting States:
**BE DE FR GB IT NL**

(56) References cited:
**US-A- 3 448 163**
**US-A- 3 641 189**

(73) Proprietor: **THE DOW CHEMICAL COMPANY**
**2030 Dow Center Abbott Road P.O. Box 1967**
**Midland Michigan 48640-1967(US)**

(72) Inventor: **Jung, Chu W.**
**34 Clark Street Apt. 7**
**Arlington Massuchusetts 02174(US)**
Inventor: **Strickler, Gary R.**
**2912 Georgetown**
**Midland Michigan 48640(US)**
Inventor: **Garrou, Philip E.**
**146 Lansdowne Road**
**Charlotte North Carolina 28226(US)**

(74) Representative: **Raynor, John et al**
**W.H. Beck, Greener & Co 7 Stone Buildings**
**Lincoln's Inn**
**London WC2A 3SZ(GB)**

**Description**

The present invention relates to the catalytic disproportionation of alkenes. Specifically, this invention relates to an improvement in the catalytic disproportionation of alkenes by use of a distillation column reactor.

According to this invention, the term disproportionation refers to the conversion of a hydrocarbon into similar hydrocarbons of both higher and lower numbers of carbon atoms per molecule. In the case of alkenes, a mixture of new products is obtained comprising alkenes of both higher and lower molecular weights. Such an operation is useful in many instances. For example, a more plentiful hydrocarbon can be converted to a less plentiful and, therefore, more valuable hydrocarbon. One instance of such a conversion occurs when the process of this invention is used to balance the alkene production of a naphtha cracking plant by disproportionating the large quantities of butenes into ethylene and hexene, or propylene and pentene. The disproportionation of butenes is a particularly valuable disproportionation reaction for the use of excess butenes. Approximately equimolar quantities of the higher and lower molecular weight alkenes may be produced by such disproportionation reactions. The higher molecular weight alkenes produced by disproportionation may be cracked to yield additional ethylene or propylene.

Much of the prior art describes conventional processes for the disproportionation of alkenes. Typically, the conventional process is carried out batchwise or in a continuous manner, using the catalyst in the form of a fixed bed, a fluidized bed or a moving bed. At the end of the reaction period, the hydrocarbon phase is separated from the solid catalyst phase and the hydrocarbon products are recovered. Well-known techniques, such as fractional distillation, solvent extraction and adsorption are employed for the separation of the hydrocarbon products.

Conventional processes for the disproportionation of alkenes, such as those described hereinabove, may be operated at temperatures as high as 500°C, but are unable to achieve simultaneous high conversion and high selectivity to desired products. See, e.g., Banks, R. L., J. Molecular Catalysis, V. 8, pp. 269-276 (1980). Alkene disproportionation reactions are reversible; therefore, theoretically the maximum conversion which can be achieved is limited by the thermodynamic equilibrium. In the disproportionation of butene-1 to ethylene and trans-hexene-3, for example, the conversion at equilibrium is approximately 50 percent. Selectivity is normally controlled by the catalyst and by process conditions, such as temperature, pressure and residence time. In a fixed bed reactor, the residence time is determined by the feed rate. In the disproportionation of butene-1, for example, a slow feed rate will result in a longer residence time for the propylene product, eventually leading to propylene disproportionation and the production of ethylene. On the other hand, a high feed rate will result in a short residence time for propylene and hence less ethylene in the product mix. Unfortunately, high feed rates reduce conversion; thus it is difficult to produce high conversion and high selectivity to propylene simultaneously. In addition, despite the numerous methods of controlling process conditions, many conventional disproportionation processes give broad product distributions, including the by-products of isomerization and secondary disproportionation reactions. Several illustrations of the prior art and its inherent limitations are presented hereinbelow.

U.S. Patent 3,261,879 (1966) discloses a disproportionation of olefin hydrocarbons by contact with a catalyst containing molybdenum oxide or tungsten oxide in a conventional reactor. Conversions are taught to vary over a wide range; however, at high conversion a broad distribution of $C_{2-12}$ olefinic products is shown. Isomerization yields are taught to be high.

U.S. Patent 3,463,827 (1969) discloses a process for the disproportionation of olefins by contact with a Group VIB metal carbonyl associated with alumina, silica or silica-alumina. The process is conducted in a fixed bed reactor, and the products are separated in a fractionation column. Conversions are taught to be low, less than 20 percent for butene-1, and isomerization tends to be high.

U.S. Patent 3,448,163 (1969) describes a conventional process for the disproportionation of olefins employing a catalyst comprising rhenium heptoxide impregnated on alumina. In the disproportionation of butene-1, the combined selectivity to ethylene and hexenes is taught to be 93 percent at a conversion of only 29 percent.

U.S. Patent 3,641,189 (1972) discloses a conventional olefin disproportionation process utilizing a rhenium heptoxide catalyst supported on alumina. High selectivities are accompanied by low conversion of the feedstock.

U.S. Patent 3,642,931 (1972) teaches a conventional olefin disproportionation process employing a catalyst comprising rhenium heptoxide supported on a refractory oxide of zirconium, thorium, tin, or mixtures thereof. The degree of disproportionation is taught to be less than 15 percent.

U.S. Patent 3,676,520 (1972) discloses a method of disproportionating olefins by contacting the olefin with a catalyst comprising rhenium oxide and a support, such as alumina. The process is carried out in any of the aforementioned standard reactors. In the disproportionation of propylene, the conversion is taught to be less than 5 percent at high contact temperature. There are no teachings on how to control the selectivities of higher olefins, such as butenes.

In view of the deficiencies of the prior art methods, it would be desirable to provide a process for the disproportionation of alkenes which would be capable of achieving simultaneous high conversion and high selectivity at moderate temperatures. It would also be desirable to provide a process for the disproportionation of butenes, such that a high yield of ethylene or propylene, and the corresponding hexenes or pentenes, could be achieved by a simple adjustment of the operating conditions. Such a process would easily meet the demands for varying olefin feedstocks. It would also be desirable to obtain the disproportionation and the separation of products simultaneously, since any reduction in the number of process steps offers considerable economic advantages.

The present invention is such a process for the disproportionation of alkenes, involving contacting at least one alkene with a catalyst in a distillation column reactor under such reaction fractionation conditions that there is formed at least one product of the disproportionation of at least one of the alkenes. By use of the distillation column reactor, this disproportionation and the separation of products are advantageously conducted simultaneously. Surprisingly, the process of the present invention proceeds with high selectivity at high conversion at moderate temperatures. Most surprisingly, the process of the present invention, as applied to butenes, can be controlled to give high yields of ethylene or propylene, whichever is desired, and the corresponding hexenes and pentenes by simple adjustments in the operating conditions. By all of the aforementioned accomplishments, the present invention satisfies a long-felt need for improvement in the art of disproportionating alkenes.

Alkenes which are subject to disproportionation according to the process of the present invention include acyclic alkenes having at least 3 carbon atoms, and their aryl derivatives and mixtures thereof. Preferred are alkenes having from 3 to 30 carbon atoms and mixtures thereof. More preferred are mono-1- and 2-alkenes, such as, for example, butene-1, and mixtures of these alkenes, such as, for example, a mixture of butene-1 and butene-2. Most preferably, the process of the present invention is applied to butene-1 or a mixture of butene-1 and butene-2. Optionally, an inert material may be included in the alkene fed to the distillation column reactor. Examples of said inert materials include nitrogen, the inert gases of Group VIIIA, such as helium and argon, and alkanes, such as methane, propane and butane.

Catalysts suitable for use in the process of the present invention are those materials which catalyze the disproportionation reaction when used in the process of the present invention, and include conventional catalysts used for the disproportionation of alkenes. Examples of said conventional catalysts include supported materials which contain catalytic metals such as rhenium, molybdenum or tungsten, and which optionally include a promoter, such as tetramethyltin or tetrabutyltin. Preferred catalysts comprise rhenium or a rhenium compound or complex having an alumina support. Rhenium oxides are preferred for use in the catalysts of the process of the present invention. The support materials can be in a variety of forms, and may contain other materials which do not substantially promote undesirable side reactions. Preferably, any conventional catalytic grade of alumina or silica-alumina may be used as the support. Gamma-alumina is the most preferred support material.

The composite catalyst is prepared by suitable methods such as dry mixing, impregnating or coprecipitation. Catalytic metal oxides or compounds convertible to catalytic metal oxides by calcination are suitably employed in the catalyst preparation. A convenient method for the preparation of the catalyst is to dry blend the catalytic metal oxide, such as rhenium oxide, and the support in a ball mill where intimate contact between the finely divided particles is achieved. The milled composite can be pressed into pellets or tablets of various sizes and shapes. Additionally, the finished catalyst may be in the form of granules as well as in other shapes, such as, for example, agglomerates, spheres and extrudates, or it may be employed in such conventional distillation packing shapes as, for example, Raschig rings and saddles. If desired, pelleted catalysts can be crushed to obtain particles having specific mesh size.

After the catalytic metal oxide or compound which may be converted to a catalytic metal oxide by calcination is associated with the support, the composite is subjected to a calcination or activation step before being utilized in the olefin conversion process. The activation technique comprises heating at elevated temperatures in the presence of a suitable flowing gas. Air is a preferred activation gas, although other gases, for example, inert gases such as nitrogen or the noble gases, may be used, provided that at least part of the catalytic metal present in the catalyst composition is in the oxide form at the completion of the activation. In some instances, the catalyst may be heated serially in more than one gas. The catalysts are subjected to a temperature which is generally in the range of from 300°C to 700°C for 0.5 to 20 hours or longer. Generally, longer activation periods are used with lower temperatures, and shorter activation periods are used with higher temperatures. Either way, the selectivities to the disproportionation products are the same.

The activated catalyst may be used, without regeneration, for runs of up to several days or more, and may be regenerated. The regeneration is accomplished by suitable methods for regenerating oxide catalysts and may comprise the same steps used in the activation procedure.

The distillation column reactor employed in the present invention is suitably a distillation column having therein a catalyst for the disproportionation of alkenes. Any type of distillation tower may be employed in the process of the present invention, provided that a fixed bed of catalyst may be created therein to fill the reaction-distillation zone or portions thereof. The catalyst packing is of such a nature as to allow vapor flow through the catalyst, while also providing sufficient surface area to catalyze the disproportionation reaction.

The distillation column reactor is operated so as to disproportionate the alkenes in the feed stream and separate the products therefrom simultaneously. Accordingly, the reactor may be operated under any conditions, e.g., temperature and pressure, at which disproportionation is achieved. Thus, the temperature within the column will be related to the boiling point of the alkene starting material, and to the pressure in the column. Typically, the operating temperature in the distillation column reactor may range from -50° C to 300° C, and preferably will be from 0° C to 150° C. The pressure in the distillation column reactor typically will be from zero to 1000 psig (6900 kPa gauge) and preferably will be from 50 to 300 psig (350 to 2100 kPa gauge). Higher or lower temperatures and pressures may be employed; however, beyond the lower end of the range, the reaction will proceed slowly, if at all, and beyond the higher end of the range, undesirable side reactions and coke formation may occur. Additionally, it will probably be more expensive to operate outside the ranges given.

The process of the present invention is a method for the simultaneous disproportionation of alkenes and distillation of the product mixture as it forms. For example, when butene-1 is fed to the distillation column reactor, it contacts the catalyst and is selectively disproportionated to ethylene and 3-hexene. The ethylene immediately upon formation ascends through the distillation column reactor, and the 3-hexene descends in accordance with conventional principles of distillation. Consequently, the reverse reaction, whereby 3-hexene and ethylene combine to form 1-butene, does not occur. Thus, in contrast to prior art disproportionation methods, which were capable of achieving high conversion with low selectivity or high selectivity at low conversion, the process of the present invention surprisingly is capable of obtaining simultaneous high conversion and high selectivity.

For the purposes of this invention, the term conversion refers to the elimination of the alkenes in the feed stream from the reaction mixture. For example, in the practice of this invention, butene-1 may be converted substantially to ethylene and hexene-3 under the proper conditions using a distillation column reactor wherein butene-1 is held in the central portion of the column and is not allowed to escape. For the purposes of the present invention, the term selectivity refers to the percentage of the converted feed which goes to the desired major products.

The concept of simultaneous high selectivity and high conversion may be expressed conveniently in terms of yield. For the purposes of the present invention, the term "yield" refers to the numerical product of conversion and selectivity (yield = conversion x selectivity). For example, a process according to the present invention operating at a conversion of 0.75 and a selectivity of 0.90 would have a yield of 0.675, which is the numerical product of 0.75 and 0.90. The process of the present invention may be operated to give higher yields than prior art disproportionation processes. Typical yields of the process of the present invention are at least 65 percent, based upon moles of alkene in the feed stream. Preferably, the yield will be at least 75 percent. Most preferably, the yield will be at least 85 percent.

A great deal of control over the rate of reaction and the distribution of products can be achieved simply by adjusting the operating conditions of the reaction system. For example, the temperature in the system may be increased by increasing the pressure, and the feed rate may be adjusted to control the percent conversion. The process of the present invention is additionally advantageous in that a great degree of control may be exercised over the percentage of propylene and ethylene produced simply by adjusting the residence time of the process. It is known that the residence time may be altered by changing any of a number of process variables, such as the feed rate, the length or height of the distillation column reactor, and the overhead temperature. When a low overhead temperature is maintained, propylene remains in contact with the catalyst for a longer time, eventually disproportionating to ethylene and butene-2. Alternatively, when a high overhead temperature is maintained, propylene leaves the column faster. Thus, for any fixed column length, the selectivity to ethylene or propylene can be controlled to a large extent by simply controlling the overhead temperature. In either case, the conversion is high in the distillation column reactor; thus, high yields of ethylene or propylene can be produced, as desired, to meet the varying olefin feedstock requirements.

The number of theoretical trays, the fractionation conditions, including temperature and pressure, the reflux and reboil control system, the number and location of side streams, the flow rate, etc., are those which are used in conventional engineering practice and can be determined by conventional design calculations and procedures. The fractionating apparatus employed in the process of the present invention may be operated using known process control techniques.

When an alkene or a mixture of alkenes is fed to a distillation column reactor under conditions previously described herein, the alkene or alkenes will be disproportionated to products having boiling points lower and higher than the boiling point of the alkene feed stream. Further, the process proceeds with high conversion and simultaneous high selectivity.

The following examples and catalyst preparations are given to illustrate the invention and should not be construed as limiting its scope. All percentages in the examples are mole percent unless otherwise indicated. Two comparative examples are given to illustrate the reaction in a conventional fixed bed reactor.

Preparation of Catalyst A

A mass (25 g) of gamma-alumina in the form of 1/8" x 1/8" (3.2 mm x 3.2 mm) tablets was added to a solution prepared by dissolving 3.35 g of $Re_2O_7$ in 250 ml of aqueous ethanol (5-10 percent $H_2O$). The resulting mixture was stirred under vacuum for a few minutes and was then evaporated to dryness on a steam bath. The resulting light grey pellets were dried at $100°C$ for 3 hours and were then calcined in a dry air stream at $600°C$ for 1 hour. Plasma emission analysis, using the 346.05 nm rhenium line, indicated a 9.04 percent rhenium loading.

Preparation of Catalyst B

A catalyst was prepared according to the method of preparation of Catalyst A except that sufficient $NH_4ReO_4$ was added to the aqueous ethanol to give a 2 percent rhenium loading. Analysis of the resulting catalyst indicated a 1.55 percent rhenium loading.

Preparation of Catalyst C

A catalyst was prepared according to the method of preparation of Catalyst A except that the catalyst was subjected to an additional calcination treatment at $500°C$ in a stream of water-saturated air (0.2 SCFH) for 2 hours. The catalyst had a 9.2 percent rhenium loading, as determined by plasma emission analysis.

Preparation of Catalyst D

A catalyst was prepared according to the method of preparation of Catalyst A except that sufficient $NH_4ReO_4$ was added to the aqueous ethanol to give a 2.5 percent rhenium loading. The catalyst was subjected to a calcination treatment at $600°C$ for 3 hours in a muffle furnace open to the air. This treatment allowed the catalyst to collect moisture from the air upon cooling. The catalyst had a 2.0 percent rhenium loading, as determined by plasma emission analysis.

Example 1

A distillation column reaction vessel was constructed from 316 stainless steel pipe. The main body of the vessel had a 3/4-inch (19 mm) outside diameter and the remainder of the vessel had a 3/8-inch (9.5 mm) outside diameter. The main body of the vessel was filled to a depth of approximately 1/2 inch (13 mm) with glass Raschig rings. Catalyst A (25 g) was added to the main body of the vessel to form a catalyst bed having an approximate size of 9 inches (230 mm) by 5/8 inch (16 mm). The remaining space in the main body of the vessel above the catalyst was filled with 1/8 inch (3.2 mm) diameter 304 stainless steel beads. The void volume of the reactor including reboiler was 70 ml, measured with the catalyst and inert packing in place. A thermosiphon reboiler was attached below the main body of the vessel. The vessel was equipped with a condensing means, means for controlling the temperature in the reboiler, means for heating the main body of the vessel, means for observing and recording the temperature at various points in the vessel, means for observing and controlling the pressure, and means for emergency relief of an overpressure condition.

Butene-1 was fed at a rate of 9.0 g per hour (60 ml/min, measured at Standard Temperature and Pressure, STP) into the reboiler of the vessel. The temperature in the reboiler was maintained at $115°C$, the temperature in the overhead condenser was maintained at $-50°C$, and the pressure in the system was

5

maintained autogenously at approximately 80 psig (550 kPa gauge). The butene-1 flashed from the reboiler into the catalyst bed where the bulk of the butene reacted at a temperature of less than 100°C. The higher boiling hexene-3 product fractionated immediately from the butene. The lower boiling ethylene product separated from the butene, passed through the condenser, and was removed from the system. The average flow rate of vapor from the top of the vessel was 30 cm³/min., measured at STP.

The overhead stream was analyzed using vapor phase chromatography and was found to be 90 mole percent ethylene and 10 mole percent propylene. The bottoms stream was analyzed using vapor phase chromatography and was found to be approximately 69.7 mole percent hexene-3, approximately 11.0 mole percent pentene, approximately 14.7 mole percent butene-1, approximately 3.7 mole percent heptene, and approximately 0.9 mole percent octene. The overall conversion was 92 mole percent, calculated according to the formula:

$$\% \text{ conversion} = \frac{\text{moles of butene-1 converted}}{\text{moles of butene-1 fed}} \times 100 \ .$$

The selectivity to the various products and by-products, based on the moles of butene-1 converted, was as follows:

| Species | Mole % Selectivity |
|---|---|
| Ethylene | 45.1 |
| Propylene | 4.9 |
| Pentenes | 6.5 |
| Hexenes | 41.3 |
| Heptenes | 2.2 |
| Octenes | 0.5 |

wherein

$$\% \text{ selectivity} = \frac{\text{moles of species formed}}{\text{moles of butene-1 converted}} \times 100 \ .$$

Absolute mole values, rather than relative mole ratios, were used in making these calculations, because the overhead stream and the bottoms stream contained different total moles of products. Thus, the yield of ethylene and hexene was approximately 79.5 percent, calculated as follows:

selectivity to ($C_2$ + $C_6$) = 0.451 + 0.413 = 0.864
conversion = 0.92
yield = 0.92 x 0.864 = 0.795.

Example 2

The method of Example 1 was repeated with the following exceptions:
(a) Catalyst B was employed (24 g);
(b) the feed rate of 1-butene was 9.6 g/hour;
(c) the reboiler temperature was 130°C; and
(d) the system pressure was maintained autogeneously at 90 psig (620 kPa gauge).
Analyses of the product streams and the selectivities, calculated as in Example 1, are shown in Table I.

6

TABLE I

| | Overhead (mole %) | Bottoms (mole %) | Selectivity (mole %) |
|---|---|---|---|
| Ethylene | 98 | - | 49 |
| Propylene | 2 | - | 1 |
| Butenes | - | 31 | - |
| Pentenes | - | 1 | 0.7 |
| Hexenes | - | 68 | 49.3 |
| Heptenes | - | - | - |
| Octenes | - | - | - |

The overall conversion was 85 mole percent and the combined yield of $C_2$ + $C_6$ hydrocarbons was approximately 84 percent.

Example 3

The method of Example 1 was repeated, with the following exceptions:
(a) Catalyst C was employed (30 g);
(b) the feed rate of 1-butene was 9.6 g/hour;
(c) the reboiler temperature was 120 °C;
(d) the overhead temperature was approximately 28 °C; and
(e) the system pressure was maintained autogeneously at 100 psig (690 kPa gauge).
Analysis of the product streams and the selectivities, calculated as in Example 1, are shown in Table II.

TABLE II

| | Overhead (mole %) | Bottoms (mole %) | Selectivity (mole %) |
|---|---|---|---|
| Ethylene | 26 | - | 17 |
| Propylene | 49 | - | 32 |
| Butenes | 24 | 40 | - |
| Pentenes | 1 | 12 | 11 |
| Hexenes | - | 41 | 36 |
| Heptenes | - | 6 | 4 |
| Octenes | - | <1 | <1 |

The overall conversion was 68 mole percent. Higher conversions may be readily attained by maintaining the overhead temperature below 0 °C to constrain the 1-butene to the reactive zone. This example demonstrates the disproportionation of butene-1 into desired $C_2$, $C_3$, $C_5$ and $C_6$ products in a yield of approximately 65 percent. Thus, as can be seen by comparing the results of Examples 1 and 3, the product mix may be varied by changing, in a simple manner, the catalyst preparation and the process operating conditions, most notably the overhead temperature.

Example 4

The method of Example 1 was repeated, with the following exceptions:
(a) Catalyst D was employed (23 g);
(b) the feed rate of 1-butene was 9.6 g/hour;
(c) the reboiler temperature was 130 °C;
(d) the overhead temperature was 0 °C; and
(e) the system pressure was maintained autogeneously at 90 psig (620 kPa gauge).
Analyses of the product streams and the selectivities, calculated as in Example 1, are shown in Table III.

7

EP 0 304 515 B1

TABLE III

|  | Overhead (mole %) | Bottoms (mole %) | Selectivity (mole %) |
|---|---|---|---|
| Ethylene | 2 | - | 1 |
| Propylene | 96 | - | 49 |
| Butenes | 2 | 14 | - |
| Pentenes | - | 35 | 20 |
| Hexenes | - | 47 | 27 |
| Heptenes | - | 3.5 | 2 |
| Octenes | - | 0.5 | 0.3 |

The overall conversion was 92 mole percent. The combined yield of $C_3$ and $C_5$ hydrocarbons was approximately 64 percent, while the combined yield of $C_2$, $C_3$, $C_5$ and $C_6$ hydrocarbons was approximately 89 percent. Thus, as can be seen by comparing the results of Examples 2 and 4, the product mix may be varied by changing the catalyst preparation in a simple manner and by changing the overhead temperature.

Comparative Experiment 1 (C.E. 1)

A catalyst comprising gamma-alumina in the form of 1/8" x 1/8" (3.2 mm x 3.2 mm) tablets and $Re_2O_7$ was prepared following the procedure described in the preparation of Catalyst A except the catalyst was calcined overnight in dry air at 530°C. Plasma emission analysis, using the 346.05 nm rhenium line indicated an 8.38 percent rhenium loading. The catalyst (25 g) was added to a vessel to form a fixed bed reactor having an approximate size of 9 inches by 5/8 inch (320 mm x 16 mm). Butene-1 was fed at a rate of 50, 150 or 200 ml/min into the reactor. The temperature of the bed was maintained at 50°C or 100°C. The pressure was maintained at 1 atmosphere in order to keep the butene in the gaseous state. The product stream was analyzed using vapor phase chromatography, and the product distribution is given in Table IV.

TABLE IV

|  |  |  |  | Selectivity (mole %) | | | |
|---|---|---|---|---|---|---|---|
| C.E.1 | Flow ml/min | Temp °C | Conv. mole % | $C_2H_4$ | $C_3H_6$ | $C_5H_{10}$ | $C_6H_{12}$ |
| (a) | 50 | 100 | 44.8 | 35.0 | 32.6 | 18.0 | 14.4 |
| (b) | 150 | 50 | 40.5 | 40.4 | 5.2 | 4.0 | 50.4 |
| (c) | 200 | 50 | 34.8 | 39.1 | 3.8 | 3.6 | 53.5 |
| (d) | 200 | 100 | 40.8 | 36.5 | 19.9 | 15.3 | 28.3 |

Comparison of Example 1 with Comparative Experiment 1(a), shows that the conversion in the fixed bed reactor is less than one-half the conversion in the distillation column reactor. Furthermore, the combined yield of ethylene and hexene, calculated as in Example 1, was only 22.1 percent in the fixed bed reactor, or less than one-third of the analogous yield in the distillation column reactor. At higher flow rates illustrated by Comparative Experiments 1(b), (c) and (d), the conversion in the fixed bed reactor decreased. Thus, in contrast to Example 1, a high conversion and high selectivity cannot be obtained simultaneously in the fixed bed reactor. Accordingly, a high yield of $C_2$ + $C_6$ hydrocarbons cannot be achieved under mild conditions.

Example 5

The method of Example 1 was repeated, with the following exceptions:
(a) the feed was an equimolar mixture of butene-1 and butene-2, with the butene-2 being 50 percent cis and 50 percent trans;
(b) the feed rate was 20 g/hour;
(c) the reboiler temperature was 130°C;
(d) the overheads temperature was approximately 5°C;
(e) the system pressure was maintained autogenously at 100 psig (690 kPa gauge); and

8

(f) the average vapor flow rate from the top of the vessel was 120 cm³/min.

Analyses of the product streams and selectivities, calculated as in Example, 1, are shown in Table V.

TABLE V

|  | Overhead (mole %) | Bottoms (mole %) | Selectivity (mole %) |
|---|---|---|---|
| Ethylene | 7 | - | 3 |
| Propylene | 92 | - | 47 |
| Butenes | 1 | 23 | - |
| Pentenes | - | 49 | 30.7 |
| Hexenes | - | 27 | 19.3 |
| Heptenes | - | - | - |
| Other | - | <1 | - |

The overall conversion was 88 mole percent and the yield to propylene and pentenes was approximately 68.4 percent. The combined yield of $C_2$, $C_3$, $C_5$ and $C_6$ hydrocarbons was 88 mole percent. Thus, it is seen that by making simple adjustments in the feedstock and the operating conditions of Example 1, the selectivity can be controlled to provide a high combined yield of $C_3$ and $C_5$ hydrocarbons.

Comparative Experiment 2 (C.E. 2)

The rhenium catalyst (8.38 percent Re) was prepared according to the procedure of Example 1, except that the catalyst was calcined at 530° C overnight in air. The fixed bed reactor of Comparative Experiment 1 was employed with 25 g of the rhenium catalyst. An equimolar mixture of butene-1 and butene-2, with butene-2 being 50 percent cis and 50 percent trans, was fed to the reactor at a rate of 20 g/hour. The temperature of the bed was maintained at 50° C, 75° C or 100° C. The pressure was maintained at 1 atmosphere so as to keep the butenes in the gaseous state. The product stream was analyzed using vapor phase chromatography and the product distribution is given in Table VI.

TABLE VI

|  |  |  | Selectivity (mole %) | | | |
|---|---|---|---|---|---|---|
| C.E.2 | Temp °C | Conv. mole % | $C_2H_4$ | $C_3H_6$ | $C_5H_{10}$ | $C_6H_{12}$ |
| (a) | 50 | 48.6 | 36.6 | 7.67 | 3.71 | 34.5 |
| (b) | 75 | 40.3 | 36.5 | 20.6 | 11.7 | 34.5 |
| (c) | 100 | 61.7 | 5.11 | 44.4 | 34.8 | 13.5 |

Comparison of Example 5 with Comparative Experiment 2(b), shows that the conversion in the fixed bed reactor is less than 50 percent of the conversion in the distillation column reactor. Furthermore, the combined yield of $C_3$ and $C_5$ hydrocarbons in the fixed bed reactor was only 13 percent, compared with 68 percent in the distillation column reactor. Even at 100° C, the combined yield of $C_3$ and $C_5$ hydrocarbons in the fixed bed reactor was only 49 percent, as shown in Comparative Experiment 2(c).

It may be noted that the conversions shown in Examples 1-5 are for a column fed at the reboiler. Conversions will be higher for a properly designed and operated distillation column reactor having the feed point located at a point in the column such that the feed alkene(s) may be retained in the catalyst bed until said alkene(s) are disproportionated.

**Claims**

1. A process for the disproportionation of alkenes, comprising contacting a composition containing at least one alkene with a disproportionation catalyst in a distillation column reactor under such reaction and fractionation conditions that there are formed products of the disproportionation of at least one of said alkenes, and such that the combined yield of said products is at least 65 mole percent.

9

2. A process according to Claim 1 wherein the catalyst contains at least one rhenium compound or complex.

3. A process according to Claim 1 or Claim 2, wherein the catalyst contains alumina.

4. A process according to Claim 3 wherein the alumina is gamma-alumina.

5. A process as claimed in any one of the preceding claims wherein the feed composition comprises (1) butene-1 or (2) butene-1 and butene-2 and the disproportionation products comprise (1) ethylene and hexene, (2) propylene and pentene, or (3) ethylene, propylene, pentene and hexene.

6. A process as claimed in any one of the preceding claims wherein the operating temperature of the distillation column reactor is in the range of from -50°C to 300°C, and the pressure is in the range of from 0 psig to 1000 psig (0 to 6900 kPa gauge).

7. A process as claimed in Claim 6 wherein the operating temperature is in the range of from 0°C to 150°C and the pressure is in the range of from 50 psig to 300 psig (350 to 2100 kPa gauge).

8. A process as claimed in any one of the preceding claims wherein the feed composition comprises butene-1; the disproportionation products comprise ethylene and hexene; the catalyst comprise $Re_2O_7$ or $NH_4ReO_4$; the pressure in the reactor is in the range of 80 psig to 90 psig (550 to 620 kPa gauge); and the temperature in the overhead condenser is -50°C.

9. A process as claimed in any one of Claims 1 to 7 wherein the feed composition comprises butene-1; the disproportionation products comprise ethylene, propylene, pentene and hexene; the catalyst comprise $Re_2O_7$ and has been calcined in a stream of water-saturated air; the pressure in the reactor is 100 psig (690 kPa gauge); and the temperature in the overhead condenser is 28°C.

10. A process as claimed in any one of Claims 1 to 7 wherein the feed composition comprises butene-1; the disproportionation products comprise propylene and pentene; the catalyst comprises $NH_4ReO_4$ and has been calcined in air; the pressure in the reactor is 90 psig (620 kPa gauge); and the temperature in the overhead condenser is 0°C.

11. A process as claimed in any one of Claims 1 to 7 wherein the feed composition comprises butene-1 and butene-2; the disproportionation products comprise propylene and pentene; the catalyst comprises $Re_2O_7$; the pressure in the reactor is 100 psig (690 kPa gauge); and the temperature in the overhead condenser is 5°C.

**Revendications**

1. Procédé de dismutation d'alcènes, comprenant la mise d'une composition, contenant au moins un alcène, en contact avec un catalyseur de dismutation, dans un réacteur à colonne de distillation, dans des conditions de réaction et de fractionnement telles qu'il se forme des produits de la dismutation d'au moins l'un desdits alcènes, et que le rendement combiné en lesdits produits en moles soit d'au moins 65 % en moles.

2. Procédé selon la revendication 1, dans lequel le catalyseur contient au moins un composé ou un complexe du rhénium.

3. Procédé selon la revendication 1 ou 2, dans lequel le catalyseur contient de l'alumine.

4. Procédé selon la revendication 3, dans lequel l'alumine est l'alumine gamma.

5. Procédé selon l'une quelconque des revendications précédentes, dans lequel la composition d'alimentation comprend (1) du butène-1 ou (2) du butène-1 et du butène-2, et les produits de dismutation comprennent (1) de l'éthylène et de l'hexène, (2) du propylène et du pentène, ou (3) de l'éthylène, du propylène, du pentène et de l'hexène.

EP 0 304 515 B1

6. Procédé selon l'une quelconque des revendications précédentes, dans lequel la température de fonctionnement du réacteur à colonne de distillation est comprise entre -500° et 300° C, et la pression manométrique est comprise entre 0 et 6,900 kPa (0 et 1000 psi).

7. Procédé selon la revendication 6, dans lequel la température de fonctionnement est comprise entre 0° C et 150° C et la pression manométrique est comprise entre 350 et 2100 kPa (50 et 300 psi).

8. Procédé selon l'une quelconque des revendications précédentes, dans lequel la composition d'alimentation comprend du butène-1 ; les produits de dismutation comprennent de l'éthylène et de l'hexène ; le catalyseur comprend $R_2O_7$ ou $NH_4ReO_4$ ; la pression manométrique dans le réacteur est comprise entre 550 et 620 kPa (80 et 90 psi) ; et la température dans le condenseur de tête vaut -50° C.

9. Procédé selon l'une quelconque des revendications 1 à 7, dans lequel la composition d'alimentation comprend du butène-1 ; les produits de dismutation comprennent de l'éthylène, du propylène, du pentène et de l'hexène ; le catalyseur comprend $Re_2O_7$ et a été calciné dans un courant d'air saturé d'eau ; la pression manométrique dans le réacteur vaut 690 kPa (100 psi) ; et la température dans le condenseur de tête vaut 28° C.

10. Procédé selon l'une quelconque des revendications 1 à 7, dans lequel la composition d'alimentation comprend du butène-1 ; les produits de dismutation comprennent du propylène et du pentène ; le catalyseur comprend $NH_4ReO_4$ et a été calciné dans l'air saturé ; la pression manométrique dans le réacteur vaut 620 kPa (90 psi) ; et la température dans le condenseur de tête vaut 0° C.

11. Procédé selon l'une quelconque des revendications 1 à 7, dans lequel la composition d'alimentation comprend du butène-1 et du butène-2 ; les produits de dismutation comprennent du propylène et du pentène ; le catalyseur comprend $Re_2O_7$ ; la pression manométrique dans le réacteur vaut 690 kPa (100 psi) ; et la température dans le condenseur de tête vaut 5° C.

**Patentansprüche**

1. Verfahren zur Disproportionierung von Alkenen, umfassend das Kontaktieren einer Zusammensetzung, die mindestens ein Alken enthält, mit einem Disproportionierungskatalysator in einem Destillationssäulenreaktor unter solchen Reaktions- und Fraktionierungsbedingungen, daß Produkte der Disproportionierung von mindestens einem der Alkene gebildet werden und daß die vereinigte Ausbeute der Produkte mindestens 65 Molprozent ist.

2. Verfahren nach Anspruch 1, worin der Katalysator mindestens eine Verbindung oder einen Komplex von Rhenium enthält.

3. Verfahren nach Anspruch 1 oder 2, worin der Katalysator Aluminiumoxid enthält.

4. Verfahren nach Anspruch 3, worin das Aluminiumoxid Gamma-Aluminiumoxid ist.

5. Verfahren nach einem der vorhergehenden Ansprüche, worin die Zufuhr-Zusammensetzung (1) 1-Buten oder (2) 1-Buten und 2-Buten enthält und die Disproportionierungsprodukte (1) Ethylen und Hexen, (2) Propylen und Penten oder (3) Ethylen, Propylen, Penten und Hexen enthalten.

6. Verfahren nach einem der vorhergehenden Ansprüche, worin die Betriebstemperatur des Destillationssäulenreaktors im Bereich von -50° C bis 300° C ist und der Druck im Bereich von 0 psig bis 1000 psig (0 bis 6900 kPa gauge) ist.

7. Verfahren nach Anspruch 6, worin die Betriebstemperatur im Bereich von 0° C bis 150° C ist und der Druck im Bereich von 50 psig bis 300 psig (350 bis 2100 kPa gauge) ist.

8. Verfahren nach einem der vorhergehenden Ansprüche, worin die Zufuhr-Zusammensetzung 1-Buten enthält, die Disproportionierungsprodukte Ethylen und Hexen enthalten, der Katalysator $Re_2O_7$ oder $NH_4ReO_4$ enthält, der Druck im Reaktor im Bereich von 80 psig bis 90 psig (550 bis 620 kPa gauge) ist und die Temperatur im Überkopfkühler -50° C ist.

11

9. Verfahren nach einem der Ansprüche 1 bis 7, worin die Zufuhr-Zusammensetzung 1-Buten enthält, die Disproportionierungsprodukte Ethylen, Propylen, Penten und Hexen enthalten, der Katalysator $Re_2O_7$ enthält und in einem Strom von wassergesättigter Luft gebrannt worden ist, der Druck im Reaktor 100 psig (690 kPa gauge) ist und die Temperatur im Überkopfkühler 28° C ist.

10. Verfahren nach einem der Ansprüche 1 bis 7, worin die Zufuhr-Zusammensetzung 1-Buten enthält, die Disproportionierungsprodukte Propylen und Penten enthalten, der Katalysator $NH_4ReO_4$ enthält und an Luft gebrannt worden ist, der Druck im Reaktor 90 psig (620 kPa gauge) ist und die Temperatur im Überkopfkühler 0° C ist.

11. Verfahren nach einem der Ansprüche 1 bis 7, worin die Zufuhr-Zusammensetzung 1-Buten und 2-Buten enthält, die Disproportionierungsprodukte Propylen und Penten enthalten, der Katalysator $Re_2O_7$ enthält, der Druck im Reaktor 100 psig (690 kPa gauge) ist und die Temperatur im Überkopfkühler 5° C ist.